# EUROPÄISCHE PATENTSCHRIFT

(11) **EP 3 182 895 B1**
(45) Veröffentlichungstag und Bekanntmachung des Hinweises auf die Patenterteilung: **30.01.2019**
(21) Anmeldenummer: 15757448.4
(22) Anmeldetag: 13.08.2015
(51) Int. Cl.: A61B 6/00

(54) **STEUERUNG DER POSITIONIERUNG EINES SCANBEREICHS EINER MEDIZINTECHNISCHEN BILDGEBENDEN ANLAGE**
CONTROL OF THE POSITIONING OF A SCANNING REGION OF A MEDICAL IMAGING SYSTEM
COMMANDE DU POSITIONNEMENT D'UNE ZONE DE BALAYAGE D'UN SYSTÈME D'IMAGERIE MÉDICALE

(30) Priorität: 22.08.2014 DE 102014216718
(43) Veröffentlichungstag der Anmeldung: 28.06.2017
(73) Patentinhaber: Siemens Healthcare GmbH, 91052 Erlangen (DE)
(72) Erfinder: UEBLER, Johann, 90411 Nürnberg (DE); WEYERMANN, Bernhard, 91315 Höchstadt (DE); HANNEMANN, Thilo, 91052 Erlangen (DE); KUHRT, Sören, 91056 Erlangen (DE)
(86) Internationale Anmeldenummer: PCT/EP2015/068627
(87) Internationale Veröffentlichungsnummer: WO 2016/026758

(56) Entgegenhaltungen:
- EP-A1- 1 382 300
- EP-A1- 2 684 522
- WO-A1-2014/033614
- DE-A1-102006 001 850
- DE-A1-102012 201 798

## Beschreibung

Die Erfindung betrifft ein Verfahren und eine Vorrichtung zur Steuerung der Positionierung bzw. Auswahl eines Scanbereichs einer medizintechnischen bildgebenden Anlage, insbesondere einer radiologischen bildgebenden Anlage wie einer Computertomographie-Anlage (CT) oder einem C-Bogen-Röntgengerät.

Vor einer bildgebenden Messung ist es in der Regel erforderlich zunächst einen sogenannten "Scanbereich" (welcher auch als Sichtbereich bzw. Field of View = FoV bezeichnet werden kann) festzulegen, d.h. den Bereich, von dem während eines "Scanvorgangs" die Bilddaten aufgenommen bzw. Rohdaten akquiriert werden, um daraus die gewünschten Bilddaten zu erzeugen. In vielen Fällen erfolgt die Festlegung des Scanbereichs mit Hilfe zumindest eines Topogramms, welches in einer einfachen Vormessung erzeugt wird. Dieses Topogramm (auch Positionierungsbild bzw. Übersichtsbild genannt) ist beispielsweise eine einfache Projektion eines Untersuchungsbereichs des Untersuchungsobjekts (z.B. der Brustkorb eines Patienten) in dem sich die für die "diagnostische" Bildgebung, bei der die für eine spätere Diagnose nutzbaren Bilddaten erzeugt werden, interessierende Region (z.B. Herz und Lunge) befindet, um damit automatisch oder manuell eine genaue Positionierung des interessierenden Bereichs vornehmen zu können. Bei einer CT-Anlage ist ein Topogramm z.B. meist eine einfache Röntgenprojektion, die in einem Scanvorgang mit bezüglich des Rotationswinkels feststehender Röntgenquelle erzeugt werden kann. Anschließend erfolgt der eigentliche Scanvorgang zur Erzeugung der dreidimensionalen Bilddaten bzw. der Schichtbilder in einer seriellen oder spiralförmigen Messung. Im Folgenden wird ohne Beschränkung der Allgemeinheit von einem Patienten als Untersuchungsobjekt ausgegangen, wobei es sich meist um einen Menschen handelt. Grundsätzlich kann der Patient aber auch ein Tier sein. Im Folgenden werden daher die beiden Begriffe "Untersuchungsobjekt" und "Patient" auch synonym verwendet.

Insbesondere bei CT-Geräten soll eine unnötige Strahlungsbelastung des Patienten vermieden werden. Daher sollte unabhängig davon, ob zunächst nur ein Scanbereich für die Aufnahme des Topogramms oder direkt ein Scanbereich für die diagnostische Bildgebung bestimmt werden muss, dieser so klein wie möglich gewählt werden. Dabei soll natürlich trotzdem die interessierende Region (ROI = Region of Interest) vollständig abgedeckt sein. Der Anwender bzw. Bediener der Anlage muss dazu genau erkennen können, welche anatomischen Regionen des Patienten von der aktuellen Einstellung erfasst werden, also ob die ROI vollständig im Scanbereich liegt. Eine fehlerhafte Einstellung führt zu unnötig an den Patienten abgegebener Röntgenstrahlung, und zwar in beiden Fällen, wenn der Scanbereich erheblich größer als die ROI ist und wenn der Scanbereich kleiner als die ROI ist, da in diesem Fall die ROI nicht ausreichend abgebildet wird und daher der Scanvorgang wiederholt werden muss.

Eine Positionierung eines Scanbereichs kann derzeit durch manuelle Auswahl einer Startlinie sowie einer Endlinie des Scanbereichs erfolgen, welche mittels eines Lichtvisiers mit Laser-Markierungslinien auf einem Patienten bzw. Untersuchungsobjekt markiert werden, der auf einem in Längsrichtung (z-Richtung) relativ zu einem Scanner (z.B. der Gantry einer CT-Anlage) verfahrbaren Objekttisch (Patientenliege) der bildgebenden Anlage liegt. Die Längsachse des Patienten ist dabei in der Regel parallel zur Längsrichtung des Objekttisches und der Objekttisch befindet sich dabei üblicherweise außerhalb des Scanners. Die Start- und Endlinie erstrecken sich dabei in Breitenrichtung (x-Richtung) des Objekttisches, wodurch der Scanbereich in Längsrichtung des Patienten definiert wird. Die Einstellung mittels des Laservisiers ist jedoch relativ zeitaufwendig. Zudem ist sicherzustellen, dass der Patient durch die Laser-Markierungslinien nicht belästigt wird.

Der Scanbereich wird durch den Nutzer nur in Richtung des Tischvorschubs eingestellt. Der Start- und Endpunkt des Scanbereichs für eine Bilddatenerfassung eines dreidimensionalen Volumens, beispielsweise bei einem CT, entspricht einer Schicht in der Bild-Rekonstruktion, die durch eine Ebene im Raum meist senkrecht zur Bewegungsrichtung des Objekttisches beim Scan repräsentiert wird.

Für den Nutzer wäre es vorteilhaft, auch die Abmessungen des Scanbereichs senkrecht zum Tischvorschub erkennen zu können, auch wenn sie nicht einstellbar sind, da sie durch die Konstruktion des CT-Scanners - insbesondere dem Abstand zwischen Röntgenquelle und -detektor und der Größe des Detektors - vorgegeben sind. Es ist dabei zwischen der Aufnahme eines Topogramms und der Aufnahme des eigentlichen CT-Scans zu unterscheiden.

Die Aufnahme eines Topogramms entspricht (in er Richtung senkrecht zum Tischvorschub) der Aufnahme einer konventionellen Röntgenaufnahme. Das optische Analogon ist die Lochkamera, wobei sich die Blende der Kamera im Fokus der Röntgenquelle befindet. Das Bildfeld wird begrenzt durch die Größe des Detektors. Während der Scanbereich in Richtung des Tischvorschubs durch zwei Ebenen begrenzt wird, die parallel zu einander sind, wird er in der Richtung senkrecht dazu durch zwei Ebenen begrenzt, die sich schneiden. Ihre Schnittlinie ist parallel zur Tischvorschubsrichtung und enthält den Fokus der Röntgenröhre. Der Öffnungswinkel der beiden Ebenen wird dadurch definiert, dass sie jeweils entgegengesetzte Enden des Detektors schneiden.

Bei der Aufnahme des eigentlichen CT-Scans werden Röntgenquelle und Detektor um den Patienten rotiert. Aus der Schnittmenge aller Topogramm-Scanbereiche ergibt sich ein kreisförmiger Bereich, der als "Field of View" (FoV) bezeichnet wird. Ein zum FoV abweichender Anzeigebereich wird algorithmisch bei der tomographischen Rekonstruktion erzeugt. Dabei ist es sehr vorteilhaft, wenn sich möglichst alle relevanten Objekte innerhalb des FoV befinden, da sich Objekte außerhalb des FoV nur sehr ungenau rekonstruieren lassen und Bildartefakte erzeugen, die sich auch auf Objekte innerhalb des FoV auswirken können.

Prinzipiell wäre es möglich, ein Bild des auf dem Objekttisch liegenden Patienten mit einer Kamera aufzunehmen und auf einem Display an einem Steuerungsterminal o. ä. der Anlage darzustellen, wobei dem Bild dann die aktuelle Start- und Endposition des Scanbereichs durch überlagerte Linien dargestellt werden können. Leider ist jedoch die Darstellung eines Start- und Endpunkts des Scanbereichs durch Linien im Bild nicht korrekt. Die Bildentstehung in der Kamera lässt sich nämlich durch ein Lochkameramodell annähern, nach dem sich alle Sichtstrahlen in einem Punkt - der Lochblende schneiden. Jedem Pixel (d.h. Bildpunkt) der Kamera ist ein Sichtstrahl zugeordnet, der die Punkte im Raum enthält, die potentiell auf diesen Pixel abgebildet werden. Jeder Pixel im Bild der Kamera entspricht dem Grau- oder Farbwert des Objekts an der Stelle, wo der durch den Pixel und die Lochblende verlaufende Sichtstrahl das Objekt schneidet. Die Sichtstrahlen aller Pixel bilden also ein divergentes Sichtstrahlenbündel.

Dies entspricht nicht der Abbildungsgeometrie des CT-Scans. Wie oben dargestellt, wird der Scanbereich in Bewegungsrichtung des Objekts durch zwei parallele Ebenen begrenzt. Die zu dieser Ebene korrespondierenden Pixel im Bild der Kamera bilden nur unter ganz bestimmten Bedingungen eine gerade Linie, nämlich im Falle einer telezentrischen Abbildung, d.h. einem (virtuell) unendlichen Abstand der Kamera vom Objekt im Lochkamera-Modell, oder wenn sich die "Lochblende" der Kamera selbst in der anzuzeigenden Raumebene befindet, d.h. direkt über der Start- bzw. Endposition. Eine telezentrische Abbildung ist konstruktiv schwer zu realisieren, da die Eintrittsoptik der Kamera mindestens Objektgröße haben müsste. Eine Kamera, die sich in der anzuzeigenden Raumebene befindet, ist dagegen offensichtlich nur für genau eine Auswahl der Raumebene realisierbar und daher prinzipiell nicht für Start- und Endposition gleichzeitig geeignet.

Aufgrund der Geometrie des Aufbaus, insbesondere der Tatsache, dass die Kamera einen großen Öffnungswinkel benötigt, um den Patienten von ihrer Position aus, z.B. an der Zimmerdecke oder oben an einer Gantry der CT-Anlage, ganz erfassen zu können, ist der entstehende Fehler nicht unerheblich und eine präzise Einstellung des Scanbereiches damit nicht mehr möglich.

Senkrecht zur Bewegungsrichtung des Objekts ergibt sich im Falle des Topogramms ein sehr ähnliches Problem mit dem Unterschied, dass die Begrenzungsebenen nicht parallel sind, sondern sich schneiden. In dem Bild der Kamera würde die seitliche Begrenzung nur dann korrekt als Linie dargestellt, wenn sich die Blende der Kamera auf der Schnittlinie der beiden Begrenzungsebenen befindet. Dies ist unpraktisch, da sich die Kamera damit sehr nah über dem Patienten befinden müsste.

Im Falle der Aufnahme eines CT-Scans ist das FoV wie oben beschrieben durch ein zylinderförmiges Volumen definiert. Dieses hat keine Entsprechung im Lochkameramodell, sondern muss über eine Berechnung im dreidimensionalen Raum abgebildet werden.

Die Dokumente WO 2014/033614 A1, EP 1 382 300 A1 und DE 10 2006 001850 A1 beschreiben jeweils Verfahren (bzw. Vorrichtungen) zur Steuerung der Positionierung eines Scanbereichs einer medizintechnischen bildgebenden Anlage für eine nachfolgende Aufnahme eines interessierenden Bereichs eines Untersuchungsobjekts, bei denen Tiefenbilddaten eines Untersuchungsobjektes erfasst werden und darauf basierend Grenzpositionen des Scanbereiches ermittelt werden.

Es ist daher eine Aufgabe der vorliegenden Erfindung, ein Verfahren und eine Vorrichtung zur Positionierung eines Scanbereichs einer medizintechnischen bildgebenden Anlage zu schaffen, um möglichst präzise und schnell, mit geringstmöglicher Belastung bzw. Belästigung eines Patienten einen Scanbereichs einstellen zu können.

Diese Aufgabe wird durch das Verfahren gemäß Patentanspruch 1 sowie durch die Vorrichtung gemäß Patentanspruch 12 gelöst.

Ein wesentlicher Verfahrensschritt des erfindungsgemäßen Verfahrens ist immer die Erfassung von Tiefenbilddaten des Untersuchungsobjekts.

Zudem werden 2D-Bilddaten zumindest eines zweidimensionalen Bilds (2D-Bild) des Untersuchungsobjekts erstellt.

Für die Nutzung dieser Daten, Tiefenbilddaten des Untersuchungsobjekts einerseits und 2D-Bilddaten andererseits gibt es im Wesentlichen zwei Grundvarianten des erfindungsgemäßen Verfahrens:
Bei einer ersten Variante wird das 2D-Bild optisch sichtbar für den Bediener der Anlage, z.B. auf einer Display-Einheit, vorzugsweise einem Touch-Screen-Monitor, dargestellt.

Die 2D-Bilddaten und die Tiefenbilddaten des Untersuchungsobjekts werden dabei zumindest bereichsweise (also beispielsweise zumindest in einem Bereich der vermuteten, später erzeugten Grenzkonturlinien des Scanbereiches und/oder an bestimmten leicht erkennbaren anatomischen Landmarken) aufeinander registriert. Vorzugsweise werden dabei die Tiefenbilddaten auf die 2D-Bilddaten registriert, so dass das 2D-Bild selbst möglichst unverändert bzw. unverzerrt bleibt. Prinzipiell können aber auch die 2D-Bilddaten auf die Tiefenbilddaten registriert werden, falls dies in einem konkreten Anwendungsfall erwünscht sein sollte. Ebenso könnte eine gegenseitige Teilregistrierung der Tiefenbilddaten und der 2D-Bilddaten zueinander erfolgen, so dass das 2D-Bild eventuell leicht verzerrt dargestellt wird, möglichst aber nicht im Scanbereich oder Untersuchungsbereich und insbesondere nicht im eigentlich interessierenden Bereich des Untersuchungsobjektes. Zur physikalisch-geometrisch korrekten bzw. verbesserten Darstellung der Start- und Endlinien des Scanbereichs sind also keine Verzerrung des Patientenbildes erforderlich. Aus anderen Gründen, z.B. zur Korrektur von Abbildungsfehlern der Optik, könnte aber trotzdem eine Verzerrung des Patientenbildes durchgeführt werden.

Unter Verwendung des dargestellten 2D-Bildes wird zumindest eine Grenzposition in Form eines oder mehrerer Punkte oder einer oder mehrerer Linien des Scanbereichs ermittelt.

Wie später noch erläutert wird, könnte hierzu der Bediener bei einer bevorzugten Variante der Erfindung zumindest einmal für die den späteren Scanvorgang beginnenden Grenzkonturlinie, im Folgenden auch als "Startlinie" des Scanbereiches bezeichnet, und einmal für die den späteren Scanvorgang beendenden Grenzkonturlinie, im Folgenden auch als "Endlinie" des Scanbereiches bezeichnet, auf einen Touch-Screen-Monitor tippen. An den Berührungsstellen auf dem Monitor können so die Grenzpositionen einer Startlinie und einer Endlinie des Scanbereiches definiert. Durch diese Punkte der Grenzpositionen hindurch geht dann der später ermittelte, insbesondere berechnete, und auf dem Monitor optisch sichtbar angezeigte Grenzverlauf des Scanbereiches.

Auf Basis der (zu den 2D-Bilddaten registrierten) Tiefenbilddaten des Untersuchungsobjekts und der Grenzposition im 2D-Bild des Untersuchungsobjekts wird dann eine durch die Grenzposition verlaufende Grenzkonturlinie ermittelt, welche (virtuell) auf einer Oberfläche, d.h. den Außenkonturen, des Untersuchungsobjekts verläuft. Diese Grenzkonturlinie wird dem 2D-Bild des Untersuchungsobjekts überlagert optisch sichtbar dargestellt, z. B. auf der besagten Display-Einheit.

Zusätzlich kann die Information über die Größe des Scanbereichs senkrecht zum Tischvorschub eingeblendet werden. Dies kann z.B. in Form einer seitlichen Begrenzungslinie erfolgen. Alternativ ist auch denkbar, dass analog zur Belichtungswarnung einer Digitalkamera, die Bereich des Patienten farblich oder blinkend hervorgehoben werden, die später nicht im Topogramm/dem CT-Scan sichtbar sein werden.

Optional wird außerdem auf Basis der Tiefenbilddaten des Untersuchungsobjekts, ein individueller dreidimensionaler Avatar für das Untersuchungsobjekt erstellt und optisch sichtbar für den Bediener der Anlage, z.B. auf einer Display-Einheit, vorzugsweise einem Touch-Screen-Monitor, telezentrisch korrekt dargestellt. Mit Hilfe der Darstellung des Avatars können dann, wie später noch erläutert wird, Grenzpositionen, bevorzugt Grenzlinien, besonders bevorzugt Grenzkonturlinien (d.h. an die Konturen des Avatars angepasste Grenzlinien) festgelegt werden, die den Scanbereich begrenzen bzw. definieren.

Senkrecht zur Tischvorschubrichtung können die Teile des Avatars markiert werden, die den Körperteilen des Patienten entsprechen, die später nicht vollständig im Topogramm/dem CT-Scan enthalten sein werden.

Unter einem Avatar ist hierbei ein künstliches virtuelles Abbild bzw. ein graphischer Stellvertreter des Patienten zu verstehen. Dieser Avatar kann auf Basis der bekannten Patientendaten individuell errechnet werden. Bevorzugt werden zumindest ein Teil, besonders bevorzugt alle, der folgenden Informationen für die Erstellung des Avatars herangezogen:
- Anatomische Landmarken zur Avatarskalierung (Beinlänge, Armlänge, Thorax-Abdomen),
- Geschlecht,
- Ethnische Merkmale,
- Körperumriss (-form),
- Körpervolumen,
- Patientenlage,
- falls bekannt, Patientengewicht.

Die wichtigsten dieser Informationen wie anatomische Landmarken, Körperumriss, Körpervolumen und Patientenlage können aus den Tiefenbilddaten ermittelt werden. Einige Informationen, wie Alter und Geschlecht, können auch aus einer elektronischen Patientenakte übernommen werden. Zumindest teilweise können aber auch ein Teil dieser Informationen besonders einfach mit Hilfe der (optional erzeugten) 2D-Bilddaten des Untersuchungsobjekts gewonnen werden. Dies gilt insbesondere für weitere anatomische Landmarken, ethnische Merkmale (die sich meist aus dem Gesicht erkennen lassen), Körperumriss (zumindest von einer Seite aus gesehen) und zum Teil die Patientenlage. Vorzugsweise werden daher auf Basis der 2D-Bilddaten weitere, insbesondere biometrische, Daten (wie z.B. Hautfarbe) des Untersuchungsobjekts ermittelt, die zur individuellen Erzeugung des Avatars für das Untersuchungsobjekt verwendet werden.

Eine erfindungsgemäße Vorrichtung zur Durchführung des Verfahrens sollte zumindest folgende Komponenten umfassen:
- Einen Tiefenbilddatensensor, welcher Tiefenbilddaten des Untersuchungsobjekts erfasst.
- Eine 2D-Kamera, wobei es sich um eine 2D-Foto-Kamera oder 2D-Video-Kamera handeln kann, welche 2D-Bilddaten für ein 2D-Bild des Untersuchungsobjekts erstellt.
- Eine Registrierungs-Einheit, welche ausgebildet ist, um die 2D-Bilddaten und die Tiefenbilddaten wie oben beschrieben zumindest bereichsweise aufeinander zu registrieren, und optional eine Avatar-Erstellungs-Einheit, welche ausgebildet ist, um auf Basis der Tiefenbilddaten, vorzugsweise unter Verwendung der 2D-Bilddaten des Untersuchungsobjekts, einen dreidimensionalen Avatar des Untersuchungsobjekts zu erstellen. Die Registrierungs-Einheit und/oder die Avatar-Erstellungs-Einheit können, z.B. in Form von Software, beispielsweise in einer Steuerungseinheit der medizintechnischen bildgebenden Anlage oder in einer Kamera-Einheit integriert sein, wie dies noch später erläutert wird.
- Eine Bild-Darstelleinheit, z.B. die Display-Einheit, um das 2D-Bild und/oder den Avatar des Untersuchungsobjekts darzustellen.
- Eine Grenzpositionierungs-Erfassungs-Einheit, welche ausgebildet ist, um unter Verwendung des 2D-Bilds und optional Avatars zumindest eine Grenzposition des Scanbereichs zu erfassen, wie dies im Zusammenhang mit dem erfindungsgemäßen Verfahren beschrieben wurde.
- Eine Grenzkonturlinien-Ermittlungs-Einheit, welche ausgebildet ist, um auf Basis der Tiefenbilddaten und der Grenzposition im 2D-Bild eine durch die Grenzposition verlaufende Grenzkonturlinie zu ermitteln, welche auf einer Oberfläche des Untersuchungsobjekts verläuft, bevorzugt so, dass die Grenzkonturlinie einem geometrischen Verlauf einer virtuell auf den Patienten projizierten Licht- bzw. Laser-Markierung entspricht. Diese Grenzkonturlinien-Ermittlungs-Einheit kann auch, z.B. als Softwaremodul, insbesondere in der Steuerungseinheit der medizintechnischen bildgebenden Anlage realisiert sein. Die erfindungsgemäße Vorrichtung ist dann zudem so ausgebildet, dass die ermittelte Grenzkonturlinie des Scanbereiches in der Bild-Darstelleinheit dem 2D-Bild überlagert dargestellt wird.

Weiterhin umfasst die Vorrichtung eine Grenzpositionierungs-Ermittlungs-Einheit, welche z.B. in der Steuerungseinheit der bildgebenden Anlage angeordnet sein kann, insbesondere auch in der Grenzpositionierungs-Erfassungs-Einheit oder der Display-Einheit. Diese Grenzpositionierungs-Ermittlungs-Einheit kann auf Basis der zuvor in der Grenzpositionierungs-Erfassungs-Einheit erfassten Positionsdaten der Grenzposition die tatsächliche Grenzposition des Scanbereichs ermitteln und diese ggfs. in einem Speicher abspeichern. Die erfassten Positionsdaten sind hierbei lediglich Sensordaten auf einem Bildschirm der Bild-Darstelleinheit. Im Gegensatz hierzu sind die ermittelten Grenzpositionsdaten ggfs. weiterverarbeitet, insbesondere um Fehler korrigiert, z.B. Auflösungsfehler auf Grund einer Fingerbreite oder unerwünschte Mehrfacheingaben mit mehreren Fingern in sehr kurzer Zeitfolge.

Weiterhin umfasst die vorliegende Erfindung eine medizintechnische bildgebende Anlage, insbesondere eine CT-Anlage, welche die erfindungsgemäße Vorrichtung enthält.

Durch die erfindungsgemäße Verwendung der 3D-Tiefenbilddaten zur perspektivisch korrekten Darstellung des Scanbereiches, indem rechnerisch perspektivisch korrigierte Grenzkonturlinien erzeugt werden, so dass sie bevorzugt geometrisch einer virtuell auf den Patienten projizierten Licht- oder Laser-Markierung entsprechen, ohne dass das angezeigte 2D-Patientenbild verzerrt wird, werden u.a. folgende Vorteile erzielt:
- Es ist eines sehr präzise Auswahl des Scanbereiches unter Berücksichtigung der aktuellen Lage des Patienten möglich.
- Es besteht "Kompatibilität" zu der bekannten Lichtvisier-Planung, da eine zum Lichtvisier analoge Darstellung der graphischen Positionslinie erfolgt. "Kompatibel" heißt in diesem Zusammenhang, dass ein Anwender bzw. Benutzer keine Schwierigkeiten bei der Nutzung der Erfindung haben sollte, da diese dem aktuell am häufigsten verwendeten Verfahren, nämlich der Positionierung eines Scanbereichs mit Laser-Markierung, zu vergleichbar ähnlichen Start- und Endlinien des Scanbereichs führt.
- Jeder vom Anwender im Patientenbild auserwählte "Orientierungspunkt" kann zur graphischen Positionsplanung erwählt werden. "Orientierungspunkte" sind bestimmte Punkte am Patienten, an denen sich der Anwender orientiert um den Scanbereich festzulegen (z.B. Ellenbogen, Kinn, Gürtelschnalle usw.). "Graphische Positionsplanung" ist eine Positionierung eines Scanbereichs eines Untersuchungsgeräts mittels Auswahl zumindest eine Start- und zumindest einer Endlinie des Scanbereichs.
- Es erfolgt ein korrekte, für den Anwender verständliche Darstellung von computer-generierten Positionsplanungen (z.B. auf Grund von Scan-Protokoll-Informationen über den Untersuchungsbereich und anatomischen Landmarken (biometrische Daten) oder anatomischen Modellen).
- Es ist eine genauere Planung ohne Strahlungsabgabe an den Patienten möglich.
- Die Darstellung des Patientenbildes auf dem Display ist möglichst realistisch und ansprechend, da keine entstellenden Bildoperationen auf dem Patientenbild nötig sind.
- Es ist kein aufwändiges Hin- und Herfahren des Tisches zur Planung mehr notwendig.

Durch die optionale Verwendung bzw. Darstellung eines Avatars, werden u.a. folgende Vorteile erzielt:
- Jeder vom Bediener im Bild mit der Darstellung des Avatars auserwählte Orientierungspunkt kann zur graphischen Positionsplanung erwählt werden. Damit ist die präzise Auswahl des Scanbereiches unter Berücksichtigung der aktuellen Lage des Patienten möglich, ohne perspektivische Verzerrungen.
- Bei dem Avatar handelt es sich um eine ansprechende Darstellung ohne fehlende Bildinformationen oder Verzerrungen, d.h. es sind auch hier keine entstellenden Bildoperationen auf dem Patientenbild nötig.
- Störende Gegenstände z. B. Infusionsflaschen oder Überwachungsgeräte, die auf einem Livebild zu sehen wären, können ausgeblendet werden.
- Bei dem Avatar handelt es sich um eine (weitgehend) anonymisierte Patientendarstellung, was die Datensicherheit erhöht. Der Patient braucht sich keine Gedanken machen, dass Kamerabilder im System gespeichert werden.
- Falls der Patient teilweise oder komplett unbekleidet auf dem Tisch liegt, kann er durch den Avatar immer angemessen repräsentiert werden.
- Eventuelle "Berührungsängste" des Bedieners, wenn er auf einem Echtzeitbild bei einem Touch-Screen "mit den Fingern" arbeiten soll, werden vermieden.
- Der Avatar kann in verschiedenen Lagen dargestellt werden, insbesondere ist eine Sicht von der Seite auf den Patienten möglich, was beispielsweise für die Einstellung einer Scan-Höhe von Interesse sein kann.

Weiterhin ist auch eine Kombination der Varianten möglich, um die jeweils für die konkrete Anwendung oder Präferenzen des Bedieners besonders interessante Vorteile der Varianten zu kombinieren. So können z.B. Grenzkonturlinien erstellt und dem dargestellten Avatar überlagert werden. Bei einer besonders bevorzugten Kombinations-Variante werden zum Beispiel die 2D-Bilddaten genutzt, um den Avatar mit einem realen Gesicht zu versehen. D.h. lediglich der Körper des Patienten wird als Avatar dargestellt und das Gesicht ist z.B. ein Live-Bild.

Bei allen Varianten kann zudem insgesamt für den Patienten die gesamte Belastung, insbesondere ggf. die Strahlenbelastung aber auch die Untersuchungszeit, minimiert werden, da das Verfahren und die Vorrichtung für den Benutzer einfacher, schneller und präzise in der Handhabung ist und die Verweildauer des Patienten in der Gantry minimiert wird. Zudem ist die Vorrichtung für den Betreiber und Hersteller kostengünstig in Herstellung, Betrieb, Wartung und Reparatur.

Die weiteren abhängigen Ansprüche sowie die nachfolgende Beschreibung enthalten besonders vorteilhafte Weiterbildungen und Ausgestaltungen der Erfindung, wobei insbesondere auch die Ansprüche einer Kategorie analog zu den abhängigen Ansprüchen einer anderen Anspruchskategorie weitergebildet sein können. Ebenso können Merkmale verschiedener Ausführungsvarianten kombiniert werden.

Die Positionsdaten der Grenzkonturlinie des Scanbereichs können entweder rechnerisch mittels Algorithmen oder mittels Auslesen von Tabellenwerten einer internen und/oder externen Datenbank ermittelt werden. Weiterhin können die Positionsdaten der Grenzkonturlinie des Scanbereichs auch durch eine Kombination der beiden Methoden ermittelt werden. Bevorzugt ist dabei die Ermittlung mit Hilfe einer Rechenvorschrift, welche die Positionsdaten der Grenzkonturlinie einem geometrischen Verlauf einer virtuell auf den Patienten projizierten Licht- oder Laser-Markierung einer senkrecht zur Oberfläche des Objekttisches verlaufenden "virtuellen Schnittfläche" zuordnet, da diese den meisten Bedienern aus dem Stand der Technik bekannt sind.

Bevorzugt wird die Grenzlinie bzw. Grenzkonturlinie des Scanbereichs in Form einer durchgezogenen oder mehrfach unterbrochenen Startlinie bzw. Endlinie dargestellt. Die virtuell auf den Patienten projizierten Licht- oder Laser-Markierungen dieser Linien verlaufen dann quasi auf der Oberfläche des Patienten in einer senkrecht zur Oberfläche des Objekttisches und Patientenlängsachse verlaufenden "virtuellen Schnittebene". Ganz besonders bevorzugt werden, wie oben bereits erwähnt, eine Startlinie und eine Endlinie erstellt, zwischen denen der Scanbereich definiert ist. Die Startlinie bzw. Endlinie sind zumindest innerhalb der seitlichen Außenumrisse des Patienten (d.h. zumindest auf dem Patienten) angezeigt. Bevorzugt ist aber die Startlinie bzw. Endlinie aus Gründen der Vereinfachung über das gesamte 2D-Bild des Patienten mit dem Objekttisch abgebildet. Die Grenz(kontur)linien verlaufen dabei vorzugsweise quer zu einer Bewegungsrichtung des Objekttisches, auf dem das Untersuchungsobjekt angeordnet ist.

In einer bevorzugten Ausführungsform der Erfindung werden die 2D-Bilddaten des Untersuchungsobjekts mit Licht im optischen Wellenlängenbereich, z.B. zwischen 380 nm und 780 nm, erstellt. Besonders bevorzugt wird das 2D-Bild als Farbbild sichtbar dargestellt. Alternativ ist auch eine Darstellung als Schwarz-Weiß-Bild mit einer Vielzahl (z.B. 256) von Graustufenwerten sinnvoll. Die 2D-Bilddaten können aber auch au-ßerhalb des optischen Wellenlängenbereichs, z.B. im Infrarot-Wellenlängenbereich zwischen 780 nm und 1000 nm, aufgenommen werden.

In einer weiter bevorzugten Ausführungsform der Erfindung werden die Tiefenbilddaten des Untersuchungsobjekts im Infrarot-Wellenlängenbereich, z.B. zwischen 780 nm und 1000 nm, erstellt, womit eine besonders robuste Ermittlung der Tiefenbilddaten möglich ist. Es können aber auch andere Wellenlängenbereiche für die Ermittlung der Tiefenbilddaten herangezogen werden.

Die 2D-Bilddaten bzw. das 2D-Bild können ein Standbild oder eine Bildfolge mit z.B. 12, 24, 50 oder 60 Bildern pro Sekunde sein. Gleiches gilt für die Tiefenbilddaten, die ebenso Standbilddaten oder Bildfolgedaten sein können.

Bevorzugt werden jeweils ein 2D-Bild sowie die zugehörigen Tiefenbilddaten des Untersuchungsobjekts, mit denen die Registrierung erfolgen soll, zeitgleich oder quasi-zeitgleich (d.h. etwa gleichzeitig, mit nur geringem Zeitabstand, z.B. von max. 1 s oder weniger) erstellt. Somit können sich unerwünschte Bewegungen des Patienten nicht in den Bilddaten niederschlagen und zu einer fehlerhaften Registrierung der 2D-Bilddaten und der Tiefenbilddaten des Untersuchungsobjekts zueinander führen.

In einer besonders bevorzugten Ausführungsform der Erfindung werden die 2D-Bilddaten und die Tiefenbilddaten des Untersuchungsobjekts aus der gleichen oder nahezu gleichen Perspektive erstellt. Z.B. haben die optischen Achsen der 2D-Bild-Kamera und des Tiefenbildsensors bevorzugt eine maximale Abweichung von ca. ± 1% einer maximalen Abmessung des Scanbereiches. Hat der Scanbereich z.B. eine Länge in z-Richtung von 0,5 m, dann beträgt die maximale Abweichung der optischen Achsen der 2D-Bild-Kamera und des Tiefenbildsensors ca. 5 mm. Eine bevorzugte Entfernung zwischen der Kamera-Einheit und dem Untersuchungsobjekt bzw. dessen geometrischer Mitte beträgt z.B. 2 m ± 50%, also zwischen 1 m und 3 m. Hiermit kann das Risiko reduziert werden, dass die Tiefenbilddaten des Untersuchungsobjekts Fehldaten auf Grund von Eigenabschattungen des Untersuchungsobjekts aufweist. Damit kann eine vollständige Ermittlung des Scanbereichs ohne ggfs. verfälschende Inter- oder Extrapolationen der Tiefenbilddaten erfolgen. Zudem wird damit einfacher verhindert, dass die Auflösung und damit die Anzahl der Pixel insbesondere in den Tiefenbilddaten zu gering ist.

In einer bevorzugten Ausführungsform der Erfindung sind hierzu die 2D-Kamera und die Tiefenbilddatensensor, besonders bevorzugt auch eine Registrierungs-Einheit, in einer gemeinsamen (d.h. räumlich vereinten) Kamera-Einheit strukturell und logisch zusammengefasst. Die Kamera-Einheit ist hierbei insbesondere bevorzugt als TOF-Kamera ("Time Of Flight"-Kamera), z.B. mit einem Photonenmischdetektor (PMD) ausgebildet, welche mittels Laufzeitverfahren mit optisch sichtbarem Licht oder Infrarot-Licht arbeiten. Solche Einheiten sind relativ kostengünstig. Alternativ kann die Kamera die 3D-Daten auch durch ein Stereobild-Verfahren oder durch Beleuchtung mit strukturiertem Licht oder eine Kombination daraus verfügbar machen. Die Kamera-Einheit kann einfach an eine Zimmerdecke oder an einen Rahmen der medizintechnischen bildgebenden Anlage montiert und mit einer Steuerungseinheit der Anlage signalleitend (drahtgebunden oder drahtlos) gekoppelt werden.

In einer besonders bevorzugten Ausführungsform der Erfindung werden die 2D-Bilddaten und die Tiefenbilddaten des Untersuchungsobjekts aus einer Perspektive erstellt, die in etwa über der geometrischen Mitte eines geplanten, voraussichtlichen Scanbereiches, zumindest in z-Richtung und optional auch in x-Richtung, angeordnet ist. D.h. die 2D-Kamera und/oder der Tiefenbildsensor sind entsprechend über dem Bereich angeordnet bzw. dorthin verfahrbar. Dies führt dazu, dass der Scanbereich etwa in zwei gleiche Teilbereiche, zumindest in z-Richtung und optional auch in x-Richtung, untergliedert ist. Damit ist zum einen das 2D-Bild weniger verzerrt und zum anderen werden Eigenabschattungen des Untersuchungsobjekts verringert und damit weisen die Tiefenbilddaten weniger zu kompensierende Fehldaten auf, jeweils im Vergleich zu einer außermittigen Positionierung der 2D-Bild-Kamera und des Tiefenbildsensors. Somit ist eine präzisere Steuerung der Positionierung des Scanbereiches möglich.

Alternativ werden in einer besonders bevorzugten Ausführungsform die 2D-Bilddaten und/oder die Tiefenbilddaten, zumindest in x-Richtung und optional auch in z-Richtung, aus einer Perspektive erstellt, die in einem Bereich über der geometrischen Mitte eine Objekttisches angeordnet ist, auf dem sich das Untersuchungsobjekt befindet, d.h. aus einer Perspektive von in etwa über der geometrischen Mitte des Untersuchungsobjekts selbst. Dies ist insbesondere von Vorteil, wenn mehrere Scanbereiche definiert werden sollen, währenddessen das Untersuchungsobjekt relativ zu einer Kamera-Einheit nicht verschoben werden soll.

In einer besonders bevorzugten Ausführungsform der Erfindung werden das 2D-Bild des Untersuchungsobjektes und/oder der Avatar sowie die Grenzlinien bzw. Grenzkonturlinien des Scanbereichs auf einer Display-Einheit mit Bild-Darstelleinheit und Linien-Positionierungs-Erfassungs-Einheit, insbesondere einem Touchscreen-Monitor (d.h. auf einem berührungsempfindlichen Bildschirm) für den Bediener optisch sichtbar dargestellt. Der Bediener kann damit wie nachfolgend beschrieben die Grenz(kontur)linie des Scanbereichs besonders einfach und intuitiv verändern. Insbesondere kann ein z.B. ohnehin bereits vorhandener Touchscreen-Monitor einer CT-Anlage zur Darstellung des 2D-Bilds des Untersuchungsobjektes sowie der Grenzkonturlinie(n) des Scanbereichs dienen.

Die Linien-Positionierungs-Erfassungs-Einheit der Display-Einheit ist dabei so ausgebildet, dass sie Bewegungen eines Zeigeobjekts (z.B. die Fingerbewegungen eines Bedieners oder Stylus-Bewegungen) vor oder auf der Display-Einheit detektiert. Es kann dann eine Grenzkonturlinie synchron mit einer Bewegungen eines Zeigeobjekts relativ zum dargestellten 2D-Bild auf der Bild-Darstelleinheit verändert werden, z.B. partiell verzerrt oder vorzugsweise in einer Bewegungsrichtung eines Objekttisches verschoben werden. Dabei wird bevorzugt immer eine Grenz(kontur)linie für eine solche Veränderung aktiviert, z.B. die, an der das Zeigeobjekt am nächsten liegt. Die Veränderung der Position der Grenz(kontur)linie des Scanbereichs relativ zum 2D-Bild des Untersuchungsobjektes ist dabei vorteilhaft durch Fingerbewegungen des Bedieners vor der Display-Einheit veränderbar. Insbesondere kann der Bediener intuitiv die Position der Grenz(kontur)linie des Scanbereichs relativ zum 2D-Bild des Untersuchungsobjektes durch Berühren der Linien-Positionierungs-Erfassungs-Einheit mit einem Finger und Verschieben des Fingers auf der Linien-Positionierungs-Erfassungs-Einheit verändern.

Dabei kann die Linien-Positionierungs-Erfassungs-Einheit auch derart ausgebildet sein, dass diese ohne Berührungen, sondern nur mit Mimik- und/oder Gestikbewegungen von Körperteilen des Bedieners im Abstand vor der Linien-Positionierungs-Erfassungs-Einheit, die Position der Grenzkonturlinie des Scanbereichs relativ zum 2D-Bild des Untersuchungsobjektes verändert. Auch eine Kombination der beiden zuvor erwähnten Möglichkeiten - mit und ohne Berührung der Linien-Positionierungs-Erfassungs-Einheit - zur Änderung der Position der Grenz(kontur)linie des Scanbereichs relativ zum 2D-Bild des Untersuchungsobjektes ist dabei möglich.

Die Erfindung wird im Folgenden unter Hinweis auf die beigefügten Figuren anhand von Ausführungsbeispielen noch einmal näher erläutert. Dabei sind in den verschiedenen Figuren gleiche Komponenten mit identischen Bezugsziffern versehen. Die Figuren sind in der Regel nicht maßstäblich. Es zeigen:
Figur 1 zwei aus der gleichen Perspektive und zeitgleich aufgenommene Abbildungen eines Patienten, links ein 2D-Bild und rechts ein Tiefenbild,
Figur 2 eine stilisierte Darstellung einer Seitenansicht in x-Richtung auf den Patienten der Figur 1, mit Darstellung einer TOF-Kamera und deren Sichtlinien in unterschiedlichen Höhen relativ zu einer Liege,
Figur 3 eine Darstellung eines auf Basis der Tiefenbilddaten erstellter Avatar eines Patienten von vorne,
Figur 4 eine Darstellung eines auf Basis der Tiefenbilddaten erstellter Avatar eines Patienten von der Seite.

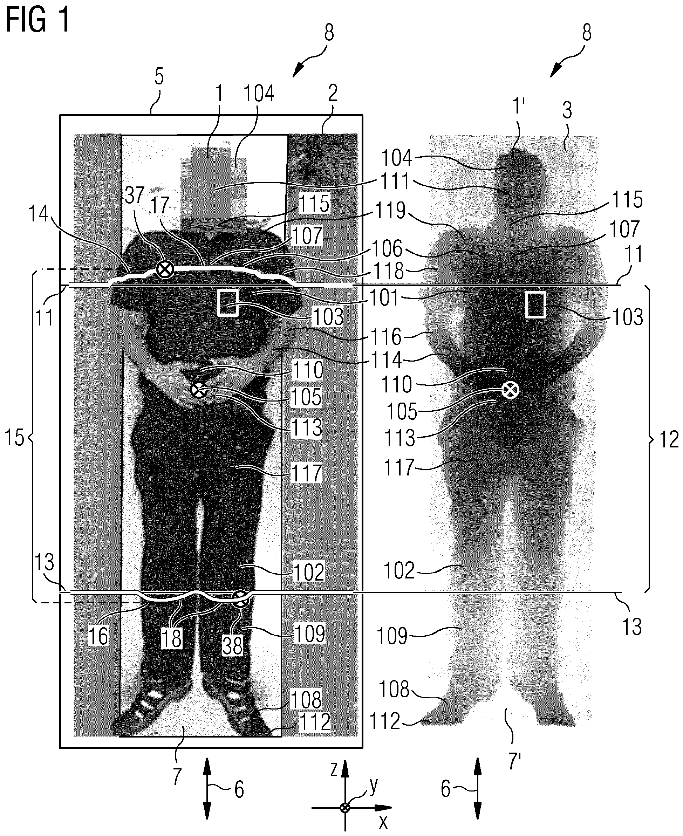

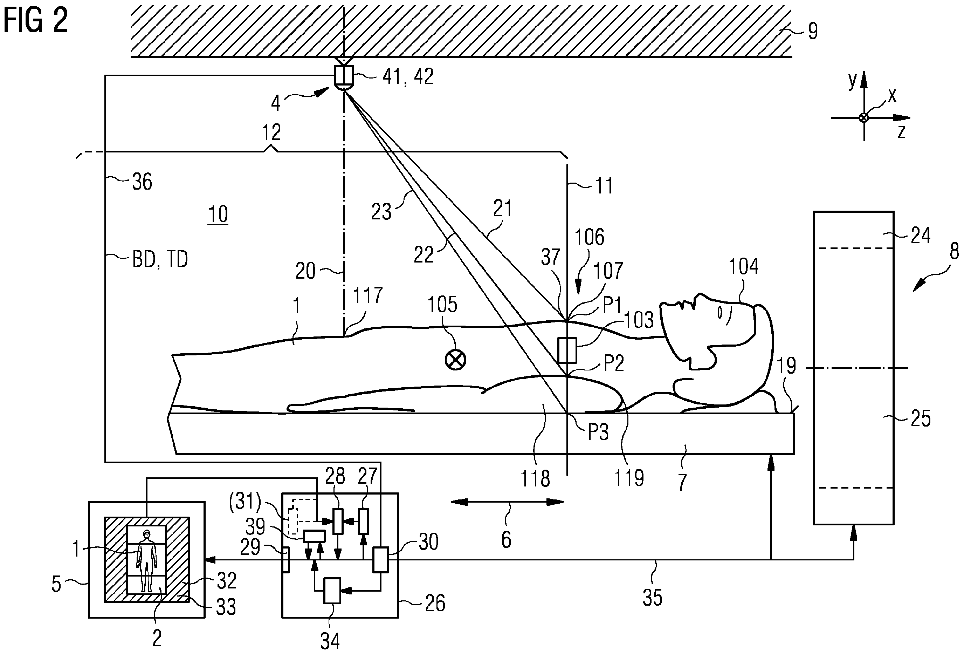

Figur 1 zeigt zwei Abbildungen eines Patienten 1, welcher wie in Figur 2 gezeigt, auf einem Objekttisch 7 (im folgenden kurz "Liege") eines nur grob schematisch dargestellten herkömmlichen CT-Gerätes 8 liegt. Die Liege 7 kann in Verfahrrichtungen 6 (in z-Richtung) verfahren werden und in einen Untersuchungsraum 25 einer Gantry 24 (auch Scanner genannt) des CT-Gerätes 8 eingefahren werden.

Links ist in Figur 1 ein dabei ein realistisches 2D-Bild 2, z.B. Photo oder Videobild, und rechts ein Tiefenbild 3 desselben Patienten 1 gezeigt. Im Tiefenbild 3 ist die Höhe mit einer Änderung von Graustufenwerten verknüpft. Die Graustufenwerte sind dort hoch, wo der Patient 1 eine große Höhe aufweist, z.B. im Brustbereich 106, im Bauchbereich 110, im Gesicht 111, im den Fußspitzenbereich 112 oder auch dort, wo die Hände 113 und Arme 114 auf dem Bauchbereich 110 aufgelegt sind. Die Graustufenwerte sind dort niedrig, wo der Patient 1 eine geringe Höhe aufweist, z.B. im Halsbereich 115, im Ellenbogenbereich 116 oder an den Unterschenkeln 109. Sowohl die 2D-Bilddaten BD des 2D-Bilds 2, als auch die Tiefenbilddaten des Tiefenbilds 3 wurden mittels einer TOF-Kamera 4 aus der gleichen Perspektive etwa zeitgleich erstellt.

Im Rahmen einer ersten Variante der Erfindung wird nur das 2D-Bild 2 auf einem Touchscreen-Monitor 5 angezeigt. In einer realen Anzeige ist dabei auch das Gesicht des Patienten erkennbar und nicht wie hier zur Anonymisierung verpixelt. Das Tiefenbild 3 ist dagegen nur aus Gründen des besseren Verständnisses der Erfindung in Figur 1 gezeigt.

Etwa 2,5 m oberhalb der Liege 7 ist die TOF-Kamera 4 z.B. an einer Decke 9 eines Untersuchungszimmers 10 angeordnet, in der sich das CT-Gerätes 8 befindet. Die Kamera 4 enthält eine digitale 2D-Kamera 41, einen digitalen Tiefenbilddatensensor 42 und ggfs. eine Registrierungs-Einheit 27 (die in diesem Fall in einer später beschriebenen Steuerungseinheit 26 entfallen könnte), wobei diese drei Bauelemente 41, 42 und 27 strukturell in dem Gehäuse der Kamera 4 integriert sind. Die 2D-Bilddaten BD des 2D-Bilds 2 wurden mit der 2D-Kamera 41 im optischen Wellenlängenbereich (380-780 nm) und die Tiefenbilddaten TD des Tiefenbilds 3 im infraroten Wellenlängenbereich (780-1000 nm) aufgenommen.

Etwas oberhalb einer Brusthöhe 101 und unterhalb der Kniegelenke 102 des Patienten 1 sind im linken 2D-Bild 2 in Breitenrichtung (x-Richtung) des Patienten 1 verlaufende, parallele obere und untere "gerade" Start- bzw. Endlinien 11, 13 mit geringer Linienbreite zwischen z.B. 1 und 10 Millimetern einzeichnet. Der Scanbereich selbst verläuft längs des Patienten 1, d.h. in der z-Richtung des Untersuchungsgerätes. Die Darstellung der geraden Start- bzw. Endlinien 11, 13 des Scanbereiches 12 erfolgt in Figur 2 nur zur besseren Erklärung der Erfindung. Sie werden vorzugsweise auf dem Monitor 5 in Wirklichkeit nicht angezeigt. Ebenso verlaufen die "geraden" Startlinien 11 und Endlinien 13 in Figur 1 auch nur aus Gründen der besseren Veranschaulichung durch das rechte Tiefenbild 3 des Patienten 1 jeweils auf gleicher Höhe in y-Richtung.

Beide "geraden" Linien 11 und 13 zeigen aber auf dem Monitor 5 Grenzen des Scanbereichs 12, die wie nachfolgend erläutert aufgrund der durch die Kamera erzeugten perspektivische Verzerrung des Patienten 1 nicht überall der realen Position der Scanbereichsgrenzen auf dem Patientenkörper entsprechen. Im linken 2D-Bild 2 des Patienten 1 sind hierzu jeweils eine entsprechende erfindungsgemäß korrekt ermittelte Startlinie 14 und Endlinie 16 des tatsächlichen Scanbereiches 15 auf dem Touchscreen-Monitor 5 eingeblendet.

Die obere Startlinie 14 und die untere Endlinie 16 wurden dabei dort positioniert, wo ein Bediener z.B. mit seinem Finger den Touchscreen-Monitor 5 zur Einstellung der jeweiligen Grenzlinie berührt hat, nämlich an einer oberen Grenzposition 37 und einer unteren Grenzposition 38. Die obere Startlinie 14 wurde durch die obere Grenzposition 37 und die untere Endlinie 16 wurde durch die untere Grenzposition 38 hindurchgeführt.

Die korrekte obere Startlinie 14 und die untere Endlinie 16 wurden auf Basis der zu den 2D-Bilddaten BD des 2D-Bilds 2 registrierten Tiefenbilddaten TD rechnerisch derart ermittelt, so dass sie jeweils möglichst genau einem geometrischen Verlauf einer virtuell auf den Patienten 1 projizierten Licht- oder Laser-Markierung entsprechen, welche in einer vertikalen Schnittebene senkrecht zur Längsachse des Patienten (y-Achse) verläuft.

Wie gut in Figur 1 zu erkennen ist, verläuft die korrekte oberen Startlinie 14 im Vergleich zur geraden Startlinie 11 in Richtung zum Kopf 104 des Patienten 1 gebogen. Der lokale Abstand zur geraden Startlinie 11 in z-Richtung ist dabei umso größer, je weiter die Startlinie 14 in Breitenrichtung (x-Richtung) des Patienten 1 zu einer geometrischen Mitte 105 des Patienten 1 des Patienten 1 verläuft, da der Patient 1 hier die größte Höhe (in y-Richtung) des Brustbereiches 106 aufweist. Hier gibt es lediglich nur eine Ausbuchtung 17 der oberen Startlinie 14, welche einen Maximalwert in der Brustmitte auf dem Brustbein 107 des Patienten 1 hat.

Die korrekte untere Endlinie 16 verläuft im Vergleich zur geraden Endlinie 13 in Richtung der Füße 108 des Patienten 1 gebogen, mit einem Abstand in z-Richtung zur geraden Endlinie 13, der umso größer ist, je weiter die Endlinie 16 in Breitenrichtung (x-Richtung) des Patienten 1 zur geometrischen Mitte 105 des jeweiligen Unterschenkels 109 des Patienten 1 verläuft, da der Patient 1 hier die beiden größten Höhen (in y-Richtung) der Unterschenkel 109 aufweist. Hier entstehen daher zwei Ausbuchtungen 18 der unteren Endlinie 16, je eine auf jedem der beiden Unterschenkel 109 des Patienten 1, deren Maximalwerte etwa in der Mitte der Unterschenkel 109 des Patienten 1 liegen.

Im Bereich der Liege 7 und des Außenumrisses des Patienten 1, fallen die geraden und die korrekten Startlinien 11, 14 und Endlinien 13, 16 jeweils aufeinander, weil die Höhen dort Null oder nur wenig mehr als Null sind, wobei die Höhe der Liege 7 als Basis "Null" in Höhenrichtung (y-Richtung) definiert ist. Deswegen ist auch die gepolsterte Liege 7 im Tiefenbild 3 nahezu gleichförmig weiß bzw. hellgrau gefärbt, da alle Bereiche der Liege 7 in etwa auf gleichem Höhenniveau liegen und nur das Polster der Liege 7 durch den darauf befindlichen Patienten 1 leicht verformt ist.

Wie der Vergleich der geraden Linien 11, 13 mit den korrekten Grenzlinien 14, 16 zeigt, gehören tatsächlich Bereiche des Patienten zum Scanbereich 15, die gemäß den geraden Linien 11, 13 nicht mehr als zum Scanbereich gehörend angezeigt würden.

Die oben definierten Start- 14 und Endlinien 16 des Scanbereiches 15 sind hier willkürlich gewählt und können natürlich gegeneinander vertauscht werden, so dass der Scanvorgang entweder vom Kopf 104 zum Fuß 108 des Patienten 1 oder alternativ von Fuß 108 zum Kopf 104 des Patienten 1 in z-Richtung verlaufen kann, und falls gewünscht auch in beiden Richtungen mit zwei Scans.

In der Seitenansicht in Figur 2 ist die Kamera 4 übrigens nur aus Gründen der deutlicheren Darstellung des Problems nicht über der geometrischen Mitte 105 des Patienten 1, sondern aus der Mitte in Richtung Beckenbereich 117 des Patienten 1 in z-Richtung versetzt angeordnet, wobei die optische Achse 20 der Kamera 4 senkrecht auf diesen Beckenbereich 117 des Patienten 1 zeigt. Die Koordinate der x-Richtung der Kamera 4 stimmt jedoch mit der x-Koordinate der geometrischen Mitte 105 etwa überein. Im Gegensatz dazu ist in Figur 1 die bevorzugte Positionierung der Kamera 4 dargestellt, wobei die optische Achse 20 der Kamera 4 in x-Richtung und auch in z-Richtung senkrecht über der geometrischen Mitte 105 im Bauchbereich 110 des Patienten 1 angeordnet ist.

In Figur 2 markiert die Startebene 11 zudem symbolisch nur den Beginn eines Scanbereiches 12, welche den Patienten 1 auf einem Längserstreckungswert in z-Richtung im Brustbereich 106 schneidet. Eine Endebene 13, 16 ist hier nicht eingezeichnet. Eine manuelle Positionierung einer End- oder Startebene kann im Übrigen auch entfallen, wobei diese dann automatisch in z-Richtung hinter den Fußspitzen 112 oder hinter dem Kopf 104 des Patienten 1 eingeblendet wird, damit der gesamte Patient 1 ab einer Startebene 11, 14 oder bis zu einer Endebene gescannt wird.

Die gerade Startebene 11 schneidet hierbei das Brustbein 107 und den linken Oberarm 118 des Patienten 1, sowie die Liege 7, wobei hierzu exemplarisch drei Schnittpunkte P1, P2 und P3 eingezeichnet sind. Der Schnittpunkt P1 ist dem Brustbein 107, der Schnittpunkt P2 der Oberfläche des linken Oberarms 118 und der Schnittpunkt P3 der Oberfläche 19 der Liege 7 zugeordnet, wobei alle drei Schnittpunkte P1, P2 und P3 in dieser Startebene 11 liegen, in derselben Entfernung von der Kamera 4. Aufgrund der unterschiedlichen Höhen der Punkte P1, P2 und P3, in y-Richtung erscheinen diese sowie die zugeordnete Sichtlinien 21, 22, 23 der Kamera 4 aber auf dem 2D-Bild 2 und dem Tiefenbilddaten TD an unterschiedlichen z-Positionen. Der Punkt P1 erscheint weiter in Richtung Kopf 104 des Patienten 1, als die Punkte P2 und P3, etwa auf einem Längenwert (in z-Richtung) der Schulter 119 des Patienten 1, während er in Wirklichkeit etwa auf gleicher Längenwert (in z-Richtung) mit der Mitte des Oberarmes 118 des Patienten 1 liegt. Entsprechend muss gemäß der Erfindung die Darstellung im 2D-Bild 2 der korrekten Startebene 14 des Scanbereiches 15 auf dem Brustbereich 106 wie oben beschrieben in Richtung Kopf 104 des Patienten 1 gewölbt sein, so dass damit die in Figur 1 dargestellten Start- 14 und Endebenen 16 des Scanbereiches 15 erzeugt wird.

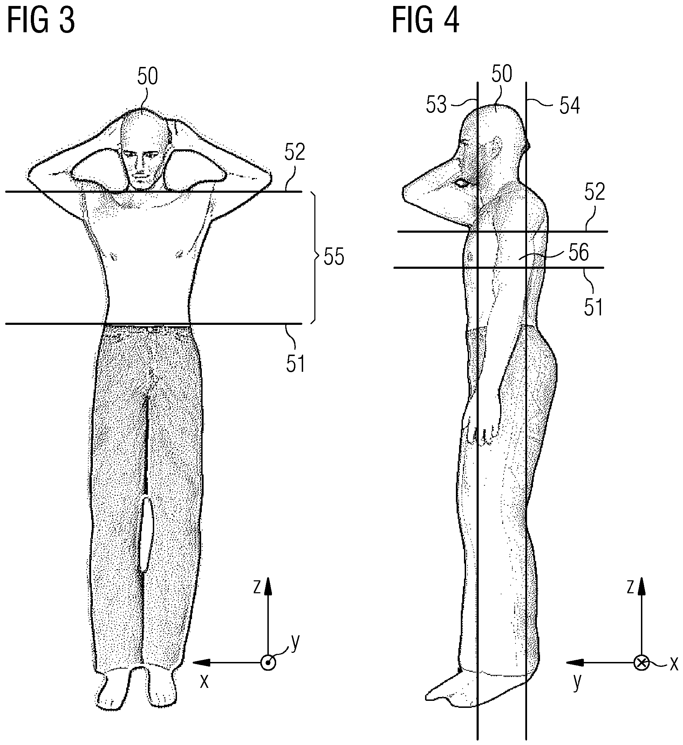

Optional wird, anstelle des vollständigen 2D-Bilds wie in Figur 1 links, ein Avatar 50 des Patienten 1 auf dem Touchscreen-Monitor 5 dargestellt. Mit Hilfe der Darstellung des Avatars 50 können dann, wie in den Figuren 3 und 4 gezeigt, Grenzlinien 51, 52, 53, 54 festgelegt werden, die den Scanbereich 55, 56 begrenzen. Dieser Avatar 50 wird auf Basis der Tiefenbilddaten TD und weiterer bekannter Patientendaten, die zum Teil auf Basis der 2D-Bilddaten BD ermittelt werden können, individuell für den Patienten 1 errechnet. Dabei werden Informationen über anatomische Größenverhältnisse, Geschlecht, ethnische Merkmale, Körperumriss etc. des Patienten 1 genutzt, um den Avatar 50 dem Patienten 1 möglichst ähnlich zu machen. Auf dem dargestellten Avatar 50 kann dann der Bediener wie nachfolgend noch erläutert wird beliebige Grenzlinien 51, 52 (z.B. eine Startlinie 51 und eine Endlinie 52) einstellen (siehe Figur 3 und 4), die den Scanbereich 55, 56 in z-Richtung begrenzen.

Neben der Darstellung von vorne wie in Figur 3 lässt sich ein solcher Avatar 50 auch drehen und in verschiedenen Lagen darstellen, so dass der Bediener sich auf dem Monitor 5 beispielsweise auch eine Sicht von der Seite auf den "Patienten" (-Avatar) anzeigen lassen kann (siehe Figur 4). Er kann so den Scanbereich 56 nicht nur in z-Richtung sondern auch in y-Richtung, bezüglich der Scan-Höhe, definieren.

In jedem Fall können die Grenzlinien 51, 52, 53, 54 als Grenzkonturlinien ausgebildet bzw. dargestellt werden, d.h. dass sie an die Kontur des Avatars 50 in einer Schnittebene (beispielsweise senkrecht oder parallel zur Liege 7) angepasst sind.

Die in Figur 2 dargestellte CT-Anlage 8 (auch CT-Gerät genannt) umfasst u.a. die nachfolgend beschriebenen Bauteile bzw. Baugruppen. Dabei wird hier lediglich als ein Beispiel davon ausgegangen, dass die CT-Anlage 8 so ausgebildet ist, dass sie die geeigneten Komponenten aufweist, um für alle oben genannten Varianten des erfindungsgemäßen Verfahrens genutzt werden zu können.

Eine ringförmige Gantry 24 mit einem mittigen Untersuchungsraum 25, sowie eine in Verfahrrichtungen 6 in den Untersuchungsraum 25 und zurück bewegbaren Liege 7 für einen Patienten 1.

Die Gantry 24 weist zumindest eine (nicht dargestellte) Röntgenstrahlenquelle sowie eine dieser relativ zum Isozentrum der Gantry 24 gegenüber liegende Röntgenstrahl-Detektoranordnung auf, wobei Röntgenstrahlenquelle und Detektoranordnung gemeinsam um das Isozentrum der Gantry 24 drehbar sind. Alternativ kann, bei ebenfalls rotierbarer Röntgenstrahlenquelle, die Detektoranordnung auch fest stehen und über zumeist 360° rings um das Zentrum der Gantry 24 angeordnet sein. Die Röntgenstrahlenquelle kann Röntgenstrahlen durch einen auf der Liege 7 im Untersuchungsraum 25 befindlichen Patienten 1 auf die Detektoranordnung strahlen, welche dann räumlich aufgelöst eine Absorption bzw. Schwächung der Röntgenstrahlen auf dem Weg durch den Körper des Patienten 1 als CT-Rohdaten bzw. Projektionsdaten detektiert.

Sowohl die Gantry 24 mit der Röntgenstrahlenquelle und der Detektoranordnung, als auch die Bewegungen der Liege 7 sind mittels eines Prozessors 30 einer Steuervorrichtung 26 über entsprechende Signalleitungen 35 steuerbar. Die mittels der Detektoranordnung erfassten CT-Rohdaten werden dann in einer CT-Bildverarbeitungs-Einheit 34 der Steuervorrichtung 26 zu CT-Bilddaten rekonstruiert und über eine Schnittstelle 29 an eine Display-Einheit 5 z.B. in Form eines Touchscreen-Monitors geleitet, der ein entsprechendes CT-Bild des Patienten 1 optisch sichtbar darstellt.

Die erfindungsgemäße Vorrichtung zur Steuerung der Positionierung des geometrischen Verlaufs eines Scanbereichs 15 beinhaltet wie beschrieben eine Kamera-Einheit 4 in Form einer TOF-Kamera, die eine 2D-Kamera 41 zur Erfassung von 2D-Bilddaten BD für 2D-Bilder 2 und einen Tiefenbilddatensensor 42 zur Erfassung von Tiefenbilddaten TD umfasst.

Weiterhin beinhaltet die erfindungsgemäße Vorrichtung eine Registrierungs-Einheit 27, eine Grenzkonturlinien-Ermittlungs-Einheit 28, eine Avatar-Erstellungs-Einheit 39 sowie eine Grenzpositionierungs-Ermittlungs-Einheit 31, welche sich allesamt in der Steuervorrichtung 26 befinden. Die Registrierungs-Einheit 27 kann wie erwähnt statt in der Steuervorrichtung 26 auch in der Kamera-Einheit 4 und die optionale Grenzpositionierungs-Ermittlungs-Einheit 31 in der Grenzpositionierungs-Erfassungs-Einheit 32 oder der Display-Einheit 5 aufgenommen sein.

Schließlich beinhaltet die erfindungsgemäße Vorrichtung au-ßerhalb der Steuervorrichtung 26 eine Grenzpositionierungs-Erfassungs-Einheit 32 und eine Bild-Darstelleinheit 33, welche beide in der Display-Einheit 5 aufgenommen sind, der beispielhaft als ein Touchscreen-Monitor 5 ausgebildet ist.

Die Bauteile bzw. Baugruppen der erfindungsgemäßen Vorrichtung sind in geeigneter Weise miteinander signalleitend verbunden, um entsprechend dem Verfahren zusammenarbeiten zu können. Dabei ist unter "signalleitend" nicht nur eine elektrisch leitende Verbindung, sondern auch eine beliebige drahtlose Verbindung zu verstehen. Insbesondere können die Bauteile bzw. Baugruppen auch über ein Bussystem untereinander verbunden sein.

Die Funktion der erfindungsgemäßen Vorrichtung ist bei der ersten Variante wie folgt:
Ein Bediener startet die Funktion des erfindungsgemäßen Verfahrens zur Steuerung der Positionierung des geometrischen Verlaufs eines Scanbereichs 15 des CT-Geräts 8 an einer (nicht dargestellten) Konsole des CT-Geräts 8. Zunächst werden 2D-Bilddaten BD des auf der Liege 7 liegenden Patienten 1 mittels 2D-Kamera 41 der TOF-Kamera 4 erstellt und diese 2D-Bilddaten BD über die Signalleitung 36 auf den Prozessor 30 der Steuerungseinheit 26 geleitet, welcher diese Bilddaten BD ggfs. nachbearbeitet und zum einen in der Registrierungs-Einheit 27 speichert und zum anderen über die Ausgangsschnittstelle 29 auf die Bild-Darstelleinheit 33 der Display-Einheit 5 leitet, wo dann diese 2D-Bilddaten BD in Form eines 2D-Bilds 2 für den Bediener dargestellt werden.

Zeitgleich mit den 2D-Bilddaten BD werden Tiefenbilddaten TD des auf der Liege 7 liegenden Patienten 1 erstellt und diese Tiefenbilddaten TD über die Signalleitung 36 auf den Prozessor 30 der Steuerungseinheit 26 geleitet, wo diese Tiefenbilddaten TD dann ggfs. nachbearbeitet und auf die Registrierungs-Einheit 27 geleitet werden. Die Tiefenbilddaten TD werden dort auf die gespeicherten 2D-Bilddaten BD registriert.

Bei Antippen der Display-Einheit 5 mit z.B. einem Finger des Bedieners, werden von Grenzpositionierungs-Erfassungs-Einheit 32 entsprechende Grenzpositionsdaten erfasst und zur Ermittlung einer Start- 14 und Endlinie 16 des Scanbereichs 16, optional über die Grenzpositionierungs-Ermittlungs-Einheit 31, an die Grenzkonturlinien-Ermittlungs-Einheit 28 geleitet.

Die Tiefenbilddaten TD werden dann aus der Registrierungs-Einheit 27 an die Grenzkonturlinien-Ermittlungs-Einheit 28 geleitet, wo zumindest ein Algorithmus, der reelle projizierte Licht- oder Laser-Markierungen virtuell wiederspiegelt, hinterlegt ist. Mittels diesem Algorithmus werden dann auf Basis der Tiefenbilddaten TD und der in die Grenzkonturlinien-Ermittlungs-Einheit 28 ebenfalls eingeleiteten Grenzpositionsdaten der Grenzpositionierungs-Erfassungs-Einheit 32 optional über die Grenzpositionierungs-Ermittlungs-Einheit 31, Grenzkonturliniendaten hier z.B. in Form einer Start- 14 und Endlinie 16 berechnet. Durch weiteres Antippen der Linien 14, 16 und Ziehen in Längsrichtung (z-Richtung) auf dem Touchdisplay, können die Positionen der Linien 14, 16 vom Bediener solange verändert werden, bis er einen passenden Scanbereich 15 zwischen den Linien 14, 16 endgültig für den nachfolgenden Scanvorgang festgesetzt hat.

Die Grenzkonturliniendaten der Start- 14 und Endlinie 16 des Scanbereichs 15 können dann in herkömmlicher Weise (wie die Positionsdaten eines Lichtvisiers) für die Steuerung des CT-Geräts bei der nachfolgenden Messung genutzt werden.

Bei einer zweiten Variante werden nach dem Start durch den Bediener die Tiefenbilddaten TD und die 2D-Bilddaten BD des auf der Liege 7 liegenden Patienten 1 mittels der TOF-Kamera 4 erstellt und an die Avatar-Erstellungs-Einheit 39 geleitet, welche unter Verwendung dieser Daten einen dreidimensionalen Avatar 50 des Untersuchungsobjekts 1 erstellt. Dieser wird dann auf der Display-Einheit 5 dargestellt.

Auch hier kann der Bediener, wie zuvor für die erste Variante beschrieben, durch Antippen der Display-Einheit 5 mit z.B. dem Finger dafür sorgen, dass entsprechende Grenzpositionsdaten bzw. Grenzlinien 51, 52, 53, 54 des Scanbereichs 55, 56 ermittelt werden. Durch weiteres Antippen der Grenzlinien 51, 52, 53, 54 und Ziehen, können auch hier die Positionen der Grenzlinien 51, 52, 53, 54 vom Bediener verändert werden, bis er einen passenden Scanbereich 55, 56 zwischen den Grenzlinien 51, 52, 53, 54 für den nachfolgenden Scanvorgang festgesetzt hat. Zusätzlich kann der Bediener hier den Avatar 50 auf der Display-Einheit 5 aktivieren und in eine beliebige Richtung drehen.

Es wird abschließend noch einmal darauf hingewiesen, dass es sich bei den vorhergehend detailliert beschriebenen Verfahren sowie bei der dargestellten Vorrichtung lediglich um Ausführungsbeispiele handelt, welche vom Fachmann in verschiedenster Weise modifiziert werden können, ohne den Bereich der Erfindung zu verlassen. Obwohl beispielsweise die Erfindung für eine Nutzung an einer Computertomographie-Anlage beschrieben wurde, schließt dies nicht die vorteilhafte Nutzung an anderen medizintechnischen bildgebenden Anlagen nicht aus, wie beispielsweise
- anderen röntgenbasierten Anlagen, z. B. zur Erstellung von herkömmliche Röntgenaufnahmen oder Durchleuchtungen;
- Magnetresonanztomographie-Geräten (MRT);
- Anlagen, um Bilder auf Basis von Radionukliden zu erstellen, z. B. Szintigraphie, Positronen-Emissions-Tomographie (PET), Single-Photon-Emissionscomputertomographie (SPECT);
- Anlagen, um Bilder auf Basis von Ultraschallwellen zu erstellen, z. B. Sonographie, Farbdoppler;
- Anlagen, um Bilder auf Basis von Infrarotstrahlung zu erstellen, z. B. diagnostische Thermographie;
- Anlagen, um Bilder auf Basis von elektrischen Widerständen bzw. Impedanzen zu erstellen, z. B. Elektrische Impedanz-Tomographie (EIT);
- Anlagen, um Bilder auf Basis von sichtbarem Licht zu erstellen, z. B. Endoskopie, optische Tomographie.

Weiterhin kann der Untersuchungsbereich grundsätzlich jede beliebige Form aufweisen, d.h. in anderen Ausführungsformen kann der Untersuchungsbereich auf dem 2D-Bild des Patienten z.B. als ein 2D-Rechteck oder 2D-Polygon oder als 2D-Freiformfläche definiert werden, anstelle durch einfache Start- und Endlinien. Weiterhin schließt die Verwendung der unbestimmten Artikel "ein" bzw. "eine" nicht aus, dass die betreffenden Merkmale auch mehrfach vorhanden sein können. Ebenso schließen die Begriff "Einheit" und "Modul" nicht aus, dass die betreffenden Komponenten aus mehreren zusammenwirkenden Teil-Komponenten bestehen, die gegebenenfalls auch räumlich verteilt sein können.

## Patentansprüche

1. Verfahren zur Steuerung der Positionierung eines Scanbereichs (15, 55, 56) einer medizintechnischen bildgebenden Anlage (8) für eine nachfolgende Aufnahme eines interessierenden Bereichs (103) eines Untersuchungsobjekts (1), wobei
- Tiefenbilddaten (TD) des Untersuchungsobjekts (1) erfasst werden,
- 2D-Bilddaten (BD) zumindest eines 2D-Bilds (2) des Untersuchungsobjekts (1) erfasst werden,
und wobei
- die 2D-Bilddaten (BD) und die Tiefenbilddaten (TD) des Untersuchungsobjekts (1) zumindest bereichsweise aufeinander registriert werden,
- das 2D-Bild (2) dargestellt wird und unter Verwendung des 2D-Bilds (2) zumindest eine Grenzposition (37, 38) des Scanbereichs (15) durch einen Bediener der Anlage definiert wird, und
- auf Basis der Tiefenbilddaten (TD) des Untersuchungsobjekts (1) und der Grenzposition (37, 38) im 2D-Bild (2) des Untersuchungsobjekts (1) eine durch die Grenzposition (37, 38) verlaufende Grenzkonturlinie (14, 16) ermittelt wird, die dem 2D-Bild (2) des Untersuchungsobjekts (1) überlagert dargestellt wird.

2. Verfahren nach Anspruch 1, das auf Basis der 2D-Bilddaten (BD) weitere, insbesondere biometrische, Daten des Untersuchungsobjekts (1) ermittelt werden, die zur individuellen Erstellung eines Avatars (50) für das Untersuchungsobjekt (1) verwendet werden.

3. Verfahren nach Anspruch 1 oder 2, wobei die Grenzkonturlinie (14, 16) einer virtuell auf das Untersuchungsobjekt (1) projizierten Licht- oder Laser-Markierung entspricht.

4. Verfahren nach einem der Ansprüche 1 bis 3, wobei zumindest zwei Grenzkonturlinien (14, 16) und/oder Grenzlinien (51, 52) des Scanbereichs (15, 55, 56) in Form einer Startlinie (14, 51) bzw. einer Endlinie (16, 52) dargestellt werden, zwischen denen der Scanbereich (15, 55, 56) definiert ist, wobei die Grenzkonturlinien (14, 16) und/oder Grenzlinien (51, 52) vorzugsweise quer zu einer Bewegungsrichtung eines Objekttisches (7) verlaufen, auf dem das Untersuchungsobjekts (1) angeordnet ist.

5. Verfahren nach einem der Ansprüche 1 bis 4, wobei die 2D-Bilddaten (BD) des Untersuchungsobjekts (1) im optischen Wellenlängenbereich erfasst werden und vorzugsweise das 2D-Bild (2) als Farbbild dargestellt wird.

6. Verfahren nach einem der Ansprüche 1 bis 5, wobei die Tiefenbilddaten (TD) des Untersuchungsobjekts (1) im Infrarot-Wellenlängenbereich erfasst werden.

7. Verfahren nach einem der Ansprüche 1 bis 6, wobei die 2D-Bilddaten (BD) und die Tiefenbilddaten (TD) des Untersuchungsobjekts (1) aus der gleichen Perspektive erfasst werden.

8. Verfahren nach einem der Ansprüche 1 bis 7, wobei die 2D-Bilddaten (BD) und/oder die Tiefenbilddaten (TD) des Untersuchungsobjekts (1) aus einer Position erfasst werden, die sich in etwa über der geometrischen Mitte eines geplanten, voraussichtlichen Scanbereiches (15) und/oder in einem Bereich über der geometrischen Mitte eines Objekttisches (7) befindet, auf dem das Untersuchungsobjekts (1) angeordnet ist.

9. Verfahren nach einem der Ansprüche 1 bis 8, wobei die 2D-Bilddaten (BD) und die Tiefenbilddaten (TD) des Untersuchungsobjekts (1) zeitgleich oder quasi-zeitgleich erstellt werden.

10. Verfahren nach einem der Ansprüche 1 bis 9, wobei das 2D-Bild (2) und/oder der Avatar sowie die Grenzkonturlinie (14, 16) und/oder Grenzlinien (51, 52, 53, 54) auf einer Display-Einheit (5) dargestellt werden, welche eine Bild-Darstelleinheit (33) und eine Linien-Positionierungs-Erfassungs-Einheit (32) aufweist.

11. Verfahren nach Anspruch 10, wobei die Linien-Positionierungs-Erfassungs-Einheit (32) der Display-Einheit (5) so ausgebildet ist, dass sie Bewegungen eines Zeigeobjekts vor und/oder auf der Display-Einheit (5) detektiert, und wobei vorzugsweise eine Grenzkonturlinie (14, 16) und/oder Grenzlinien (51, 52, 53, 54) synchron mit einer Bewegungen eines Zeigeobjekts relativ zum dargestellten 2D-Bild (2) auf der Bild-Darstelleinheit (33) verändert wird, besonders bevorzugt in einer Bewegungsrichtung eines Objekttisches (7) verschoben wird, auf dem das Untersuchungsobjekts (1) angeordnet ist.

12. Vorrichtung zur Steuerung der Positionierung eines Scanbereichs (15) einer medizintechnischen bildgebenden Anlage (8) für eine nachfolgende Aufnahme eines interessierenden Bereichs (103) eines Untersuchungsobjekts (1), wobei die Vorrichtung umfasst:
- einen Tiefenbilddatensensor (42), welcher Tiefenbilddaten (TD) des Untersuchungsobjekts (1) erfasst,
- eine 2D-Kamera (41), welche 2D-Bilddaten (BD) zumindest eines 2D-Bilds (2) des Untersuchungsobjekts (1) erstellt,
- eine Registrierungs-Einheit (27), welche die 2D-Bilddaten (BD) und die Tiefenbilddaten (TD) des Untersuchungsobjekts (1) zumindest bereichsweise aufeinander registriert,
- eine Bild-Darstelleinheit (33), welche das 2D-Bild (2) des Untersuchungsobjekts (1) darstellt,
- eine Grenzpositionierungs-Erfassungs-Einheit (32), welche unter Verwendung des dargestellten 2D-Bilds (2) die Eingabe zumindest einer Grenzposition (37, 38, 51, 52) des Scanbereichs (15, 55, 56) durch einen Bediener der Anlage erfasst, und
- eine Grenzkonturlinien-Ermittlungs-Einheit (28), welche auf Basis der Tiefenbilddaten (TD) und der Grenzposition (37, 38) des Scanbereichs (15) im 2D-Bild (2) eine durch die Grenzposition (37, 38) verlaufende Grenzkonturlinie (14, 16) des Scanbereichs (15) ermittelt, die in der Bild-Darstelleinheit (33) dem 2D-Bild (2) überlagert dargestellt wird.

13. Vorrichtung nach Anspruch 12, wobei die 2D-Kamera (41), und der Tiefenbilddatensensor (42) und vorzugsweise die Registrierungs-Einheit (27) zu einer Kamera-Einheit (4) zusammengefasst sind.

14. Vorrichtung nach Anspruch 12 oder 13, wobei die Bild-Darstelleinheit (33) und die Linien-Positionierungs-Erfassungs-Einheit (32) in einer Display-Einheit (5) zusammengefasst sind, insbesondere in einem Touch-Screen-Monitor (5) .

15. Medizintechnische bildgebende Anlage (8), insbesondere Computertomographiegerät, enthaltend die Vorrichtung nach einem der Ansprüche 12 bis 14.

## Claims

1. Method for controlling the positioning of a scanning region (15, 55, 56) of a medical imaging system (8) for subsequent recording of a region of interest (103) of an examination object (1), wherein
- depth image data (TD) of the examination object (1) is captured,
- 2D image data (BD) of at least one 2D image (2) of the examination object (1) is captured,
and wherein
- the 2D image data (BD) and the depth image data (TD) of the examination object (1) are registered to each other at least in some regions,
- the 2D image (2) is displayed and, by using the 2D image (2) at least one limit position (37, 38) of the scanning region (15) is defined by an operator of the system and
- a limit contour line (14, 16) extending through the limit position (37,38) is determined on the basis of the depth image data (TD) of the examination object (1) and the limit position (37, 38) in the 2D image (2) of the examination object (1), said limit contour line being displayed superimposed on the 2D image (2) of the examination object (1).

2. Method according to claim 1, the further, in particular biometric, data of the examination object (1) is determined on the basis of the 2D image data (BD), said data being used for the individual creation of an avatar (50) for the examination object (1).

3. Method according to claim 1 or 2, wherein the limit contour line (14, 16) corresponds to a light or laser marking projected virtually onto the examination object (1).

4. Method according to one of claims 1 to 3, wherein at least two limit contour lines (14, 16) and/or limit lines (51, 52) of the scanning region (15, 55, 56) are displayed in the form of a start line (14, 51) or an end line (16, 52) between which the scanning region (15, 55, 56) is defined, wherein the limit contour lines (14, 16) and/or limit lines (51, 52) preferably extend transversely to a direction of movement of an object table (7) on which the examination object (1) is arranged.

5. Method according to one of claims 1 to 4, wherein the 2D image data (BD) of the examination object (1) is captured in the optical wavelength range and the 2D image (2) is preferably displayed as a colour image.

6. Method according to one of claims 1 to 5, wherein the depth image data (TD) of the examination object (1) is captured in the infrared wavelength range.

7. Method according to one of claims 1 to 6, wherein the 2D image data (BD) and the depth image data (TD) of the examination object (1) are captured from the same perspective.

8. Method according to one of claims 1 to 7, wherein the 2D image data (BD) and/or the depth image data (TD) of the examination object (1) are captured from a position, which is located approximately over the geometric centre of a planned, envisaged scanning region (15) and/or in a region over the geometric centre of an object table (7) on which the examination object (1) is arranged.

9. Method according to one of claims 1 to 8, wherein the 2D image data (BD) and the depth image data (TD) of the examination object (1) are created simultaneously or quasi-simultaneously.

10. Method according to one of claims 1 to 9, wherein the 2D image (2) and/or the avatar and the limit contour line (14, 16) and/or limit lines (51, 52, 53, 54) are displayed on a display unit (5) comprising an image-display unit (33) and a line-positioning-capturing unit (32).

11. Method according to claim 10, wherein the line-positioning-capturing unit (32) of the display unit (5) is embodied such that it detects movements of a pointer object before and/or on the display unit (5) and wherein preferably a limit contour line (14, 16) and/or limit lines (51, 52, 53, 54) is changed synchronously with a movement of a pointer object relative to the 2D image (2) displayed on the image-display unit (33), particularly preferably displaced in a direction of movement of an object table (7) on which the examination object (1) is arranged.

12. Device for controlling the positioning of a scanning region (15) of a medical imaging system (8) for subsequent recording of a region of interest (103) of an examination object (1), wherein the device comprises:
- a depth-image-data sensor (42), which captures depth image data (TD) of the examination object (1),
- a 2D camera (41), which creates 2D image data (BD) of at least one 2D image (2) of the examination object (1),
- a registration unit (27), which registers the 2D image data to each other (BD) and the depth image data (TD) of the examination object (1) at least in some regions,
- an image-display unit (33), which displays the 2D image (2) of the examination object (1),
- a limit-positioning-capturing unit (32), which captures the input of at least one limit position (37, 38, 51, 52) of the scanning region (15, 55, 56) by an operator of the system by using the 2D image (2) displayed and
- a limit-contour-line-determining unit (28), which determines a limit contour line (14, 16) of the scanning region (15) extending through the limit position (37, 38) on the basis of the depth image data (TD) and the limit position (37, 38) of the scanning region (15) in the 2D image (2), said limit contour line being displayed superimposed on the 2D image (2) in the image-display unit (33).

13. Device according to claim 12, wherein the 2D camera (41) and the depth-image-data sensor (42) and preferably the registration unit (27) are combined to form a camera unit (4).

14. Device according to claim 12 or 13, wherein the image-display unit (33) and the line-positioning-capturing unit (32) are combined in a display unit (5), in particular in a touch-screen monitor (5).

15. Medical imaging system (8), in particular a computed tomography device containing the device according to one of claims 12 to 14.

## Revendications

1. Procédé de commande du positionnement d'une zone (15, 55, 56) de balayage d'un système (8) d'imagerie médicale pour une prise de vue ultérieure d'une région (103) à laquelle on s'intéresse d'un objet (1) à examiner, dans lequel
- on relève des données (TD) d'image en profondeur de l'objet (1) à examiner,
- on relève des données (BD) d'image en 2D d'au moins une image (2) en 2D de l'objet (1) à examiner,
et dans lequel
- on enregistre, les unes sur les autres au moins par zone, les données (BD) d'image en 2D et les données (TD) d'image en profondeur de l'objet (1) à examiner,
- on représente l'image (2) en 2D et, en utilisant l'image (2) en 2D, on définit au moins une position (37, 38) limite de la zone (15) de balayage par un opérateur du système et
- sur la base des données (TD) d'image en profondeur de l'objet (1) à examiner et de la position (37, 38) limite dans l'image (2) en 2D de l'objet (1) à examiner, on détermine une ligne (14, 16) de contour limite passant par la position (37, 38) limite, que l'on représente superposée à l'image (2) en 2D de l'objet (1) à examiner.

2. Procédé suivant la revendication 1, suivant lequel on détermine, sur la base des données (BD) d'image en 2D, d'autres données, notamment biométriques, de l'objet (1) à examiner, que l'on utilise pour l'établissement individuel d'un avatar (50) de l'objet (1) à examiner.

3. Procédé suivant la revendication 1 ou 2, dans lequel la ligne (14, 16) de contour limite correspond à un repérage lumineux ou laser projeté virtuellement sur l'objet (1) à examiner.

4. Procédé suivant l'une des revendications 1 à 3, dans lequel on représente au moins deux lignes (14, 16) de contour limite et/ou des lignes (51, 52) limites de la zone (15, 55, 56) de balayage, sous la forme d'une ligne (14, 51) initiale ou d'une ligne (16, 52) finale, entre lesquelles la zone (15, 55, 56) de balayage est définie, les lignes (14, 16) de contour limites et/ou les lignes (51, 52) limites, s'étendant de préférence transversalement à une direction de déplacement d'une table (7) d'objet, sur laquelle l'objet (1) à examiner est mis.

5. Procédé suivant l'une des revendications 1 à 4, dans lequel on relève les données (BD) d'image en 2D de l'objet (1) à examiner dans le domaine des longueurs d'ondes optiques et on représente, de préférence, l'image (2) en 2D sous la forme d'une image en couleur.

6. Procédé suivant l'une des revendications 1 à 5, dans lequel on relève les données (TD) d'image en profondeur de l'objet (1) à examiner dans le domaine des longueurs d'ondes infrarouges.

7. Procédé suivant l'une des revendications 1 à 6, dans lequel on relève les données (BD) d'image en 2D et les données (TD) d'image en profondeur de l'objet (1) à examiner à partir de la même perspective.

8. Procédé suivant l'une des revendications 1 à 7, dans lequel on relève les données (BD) d'image en 2D et/ou les données (TD) d'image en profondeur de l'objet (1) à examiner à partir d'une position, qui se trouve à peu près au milieu géométrique d'une zone (15) de balayage probable prévue et/ou dans une zone au milieu géométrique d'une table (7) d'objet, sur laquelle l'objet (1) à examiner est mis.

9. Procédé suivant l'une des revendications 1 à 8, dans lequel on produit les données (BD) d'image et les données (TD) d'image en profondeur de l'objet (1) à examiner en même temps ou quasiment en même temps.

10. Procédé suivant l'une des revendications 1 à 9, dans lequel on représente l'image (2) en 2D et/ou l'avatar ainsi que la ligne (14, 16) de contour limite et/ou les lignes (51, 52, 53, 54) limites sur une unité (5) d'affichage, qui a une unité (33) de représentation d'image et une unité (32) de détection du positionnement des lignes.

11. Procédé suivant la revendication 10, dans lequel l'unité (32) de détection de positionnement des lignes de l'unité (5) d'affichage est constituée de manière à détecter des déplacements d'un objet (5) indicateur, devant et/ou sur l'unité (5) d'affichage, et dans lequel on modifie, de préférence, une ligne (14, 16) de contour limite et/ou des lignes (51, 52, 53, 54) limites en synchronisme avec un déplacement d'un objet indicateur par rapport à l'image (2) en 2D représentée sur l'unité (33) de représentation d'image, en la déplaçant d'une manière particulièrement préférée dans une direction de déplacement d'une table (7) d'objet, sur lequel l'objet (1) à examiner est mis.

12. Installation de commande du positionnement d'une zone (15) de balayage d'un système (8) d'imagerie médicale pour une prise de vue ultérieure d'une zone (103) à laquelle on s'intéresse d'un objet (1) à examiner, l'installation comprenant :
- un capteur (42) de données d'image en profondeur, qui relève des données (TD) d'image en profondeur de l'objet (1) à examiner,
- un appareil photographique (41) en 2D, qui produit des données (BD) d'image en 2D d'au moins une image (2) en 2D de l'objet (1) à examiner,
- une unité (27) d'enregistrement, qui enregistre les données (BD) d'image en 2D et les données (TD) d'image en profondeur de l'objet (1) à examiner, les unes sur les autres au moins par zone,
- une unité (3) de représentation d'image, qui représente l'image (2) en 2D de l'objet (1) à examiner,
- une unité (32) de détection en positionnement de limite, qui, en utilisant l'image (2) en D2 représentée, relève l'entrée d'au moins une position (37, 38, 51, 52) limite de la zone (15, 55, 56) de balayage par un opérateur du système et
- une unité (28) de détermination de ligne de contour limite, qui, sur la base des données (TD) d'image en profondeur et de la position (37, 38) limite de la zone (15) de balayage dans l'image (2) en 2D, détermine une ligne (14, 16) de contour limite, passant par la position (37, 38) limite, de la zone (15) de balayage, qui est représentée dans l'unité (33) de représentation d'image en étant superposée à l'image (2) en 2D.

13. Installation suivant la revendication 12, dans laquelle l'appareil (41) photographique en 2D et le capteur (42) de données d'image en profondeur et de préférence l'unité (27) d'enregistrement sont rassemblés en une unité (4) d'appareil photographique.

14. Installation suivant la revendication 12 ou 13, dans laquelle l'unité (33) de représentation d'image et l'unité (32) de détection de positionnement des lignes sont rassemblées en une unité (5) d'affichage, notamment en un touch screen monitor (5).

15. Système (8) d'imagerie médicale, notamment appareil de tomographie à ordinateur, comportant le système suivant l'une des revendications 12 à 14.
